# EUROPÄISCHE PATENTANMELDUNG

(11) **EP 2 147 904 A2**
(43) Veröffentlichungstag der Anmeldung: **27.01.2010**
(21) Anmeldenummer: 09165534.0
(22) Anmeldetag: 15.07.2009
(51) Int. Cl.: C04B 35/64, A61C 13/00, A61K 6/06

(54) **Verfahren zum dimensionstreuen Sintern eines Formteils**

(30) Priorität: 18.07.2008 DE 102008002952
(71) Anmelder: DeguDent GmbH, 63457 Hanau (DE)
(72) Erfinder: Kutzner, Martin, 63543 Neuberg (DE); Tullney, Sarah, 64297 Darmstadt (DE)
(74) Vertreter: Stoffregen, Hans-Herbert

(57) **Zusammenfassung**

Die Erfindung bezieht sich auf ein Verfahren zum dimensionstreuen Sintern eines aus einem aus porösem keramischen Material bestehenden Rohling hergestellten Formteils (10), , wobei das Formteil während des Sinterns über zumindest eine erste Verbindung (20, 22, 24) mit einem Träger (14) verbunden bleibt, der aus dem Rohling hergestellt wird. Um mit einfachen Verfahrensschritten und konstruktiv einfachen Maßnahmen sicherzustellen, dass beim Sintern des aus dem porösem keramischen Material bestehenden Formteils eine Geometrieveränderung vermieden wird, ist erfindungsgemäß vorgesehen, dass der Träger (14) als ein das Formteil (10) zumindest bereichsweise umlaufend umgebender Abschnitt des Rohlings durch materialabtragende Bearbeitung aus dem Rohling hergestellt wird, wobei nach der Herstellung der Träger zum einen über die zumindest eine erste Verbindung (20, 22, 24, 34, 36) mit dem Formteil und zum anderen über zumindest eine zweite Verbindung (16, 18, 30, 32) mit dem den Träger abschnittsweise beabstandet umgebenden Restrohling (12) verbunden ist.

## Beschreibung

Die Erfindung bezieht sich auf ein Verfahren zum dimensionstreuen Sintern eines aus einem aus porösem keramischen Material bestehenden Rohling hergestellten Formteils, insbesondere einer zahntechnischen Rekonstruktion, wobei das Formteil während des Sinterns über zumindest eine erste Verbindung, wie Steg, mit einem Träger verbunden bleibt, der aus dem Rohling hergestellt wird. Auch nimmt die Erfindung Bezug auf einen Restrohling als Teil eines aus porösem keramischen Material bestehenden Rohlings mit einem aus dem Rohling durch Materialabtragung hergestellten Formteil, das integral mit dem Restrohling verbunden ist.

Ein Verfahren der eingangs genannten Art ist aus der EP-B-1 154 969 bekannt. Um zu verhindern, dass beim Sintern des Formteils, das aus einem keramischen Werkstoff besteht, aufgrund der auftretenden Volumenreduzierung (Schwund) eine Geometrieveränderung durch Relativbewegung zwischen dem zu sinternden Formteil und einer diesen abstützenden Unterlage erfolgt, ist vorgesehen, dass das Formteil auf einem beweglichen Träger wie Haltesteg oder auf Rollen abgestützt ist. Auch besteht die Möglichkeit, das Formteil und von diesem integral ausgehende Stege mit einer Brennunterlage zu verbinden, die gleichfalls zusammen mit den Formteilen aus einem Rohling durch Materialabtrag hergestellt ist. Bei einer mehrgliedrigen Brücke geht von jedem Brückenglied ein Steg aus.

Hierdurch ist der Nachteil gegeben, dass bei einem Verformen der Unterlage die Geometrieänderung nur auf eines der Glieder übertragen werden kann, wodurch eine Relativbewegung zwischen den Gliedern und damit eine unerwünschte Formveränderung auftreten kann.

Die WO-A-99/47065 und WO-A-02/45614 beziehen sich auf Zahnersatzteile, die aus porösem keramischen Material bestehenden Rohlingen durch Sintern hergestellt werden. Hierzu wird der Rohling von einem Rahmen aufgenommen, um durch fräsende Bearbeitung den Zahnersatz herzustellen. Der Zahnersatz wird dabei zunächst über Stege mit dem von dem Rohling verbleibenden Restrohling verbunden, die zum Schluss der Verarbeitung abgetrennt werden. Die Trennstellen werden sodann glatt geschliffen. Anschließend erfolgt das Sintern.

Aus der WO-A-2005/051220 ist ein Verfahren zur Herstellung eines Käppchens als Formteil bekannt. Dabei wird das Formteil aus einem Rohling herausgearbeitet, wobei vor dem Lösen des Formteils aus dem Rohling dieser mit dem Restrohling über einen oder mehrere Stege verbunden bleibt, der bzw. die anschließend durchtrennt werden. Sodann erfolgt ein Durchsintern.

Der vorliegenden Erfindung liegt die Aufgabe zugrunde, ein Verfahren sowie eine zu sinternde Einheit der eingangs genannten Art so weiterzubilden, dass mit einfachen Verfahrensschritten und konstruktiv einfachen Maßnahmen sichergestellt ist, dass beim Sintern des aus dem porösem keramischen Material bestehenden Formteils eine Geometrieveränderung nicht auftritt.

Verfahrensmäßig wird die Aufgabe im Wesentlichen dadurch gelöst, dass der Träger als ein das Formteil zumindest bereichsweise umlaufend umgebender Abschnitt des Rohlings durch materialabtragende Bearbeitung hergestellt wird, wobei der Träger zum einen über die zumindest eine erste Verbindung mit dem Formteil und zum anderen über zumindest eine zweite Verbindung mit dem den Träger zumindest abschnittsweise beabstandet umgebenden Restrohling verbunden ist.

Insbesondere ist vorgesehen, dass der Träger derart durch materialabtragende Bearbeitung, insbesondere durch Fräsen des Rohlings hergestellt wird, dass der Träger umlaufend das Formteil umgibt und derart aus dem Rohling herausgearbeitet wie herausgefräst wird, dass der Träger zum einen über eine erste Membran und/oder über zumindest zwei erste Stege mit einem Formteil und zum anderen mit dem verbleibenden Restrohling über eine zweite Membran und/oder über zumindest zwei zweite Stege verbunden ist.

Abweichend vom Stand der Technik wird das zu sinternde Formteil von dem Träger vorzugsweise umlaufend umgeben und mit diesem zusammen gesintert, wobei der Träger als Abstützung während des Sinterns dient. Hierdurch wird sichergestellt, dass eine dimensionstreue Volumenreduzierung erfolgt, ohne dass geometrieverfälschende Änderungen am Formteil auftreten. Dabei dient erwähntermaßen vorzugsweise der Träger selbst als Abstützung auf einer Unterlage. Hierzu ist vorgesehen, dass das Formteil zurückversetzt zu zumindest einem Abschnitt einer Außenfläche des Trägers verläuft, auf dem der Restrohling beim Sintern auf einer Unterlage positioniert wird.

Die Erfindung wird jedoch auch dann nicht verlassen, wenn beim Sintern der Träger zusammen mit dem Formteil und dem Restrohling verbunden bleibt, so dass die diesbezügliche Einheit insgesamt durchgesintert wird. Auch bei diesen Maßnahmen ist sichergestellt, dass eine dimensionstreue Volumenreduzierung erfolgt, ohne dass Geometrieverfälschungen erfolgen; denn der Restrohling ist mit dem Träger über zweite Verbindungen verbunden, die vorzugsweise punktuell z. B. durch Stege ausgebildet sind.

Es besteht jedoch auch die Möglichkeit, dass die Verbindung zwischen dem Formteil und dem Träger und/oder die Verbindung zwischen dem Träger und dem Rohling in Form von einer umlaufenden Membran oder durch Abschnitte einer Membran gebildet wird. Durch die hierdurch bedingte Materialverdünnung zwischen den gegeneinander abgestützten Elementen ist gleichfalls sichergestellt, dass ein dimensionstreues Durchsintern des Formteils erfolgen kann.

Unabhängig hiervon ist vorgesehen, dass der Träger und das Formteil über mehrere gleichmäßig über die Außenumfangsfläche des Formteils verlaufende erste Verbindungen wie Stege verbunden sind. Der Träger ist gleichfalls über mehrere vorzugsweise stegförmige zweite Verbindungen mit dem Restrohling verbunden, wobei die zweiten Verbindungen ebenfalls gleichmäßig oder im Wesentlichen gleichmäßig über den Umfang verteilt sein sollten.

Des Weiteren sollten die ersten und zweiten Verbindungen versetzt zueinander angeordnet sein. Dabei kann die Anzahl der zweiten Verbindungen geringer als die der ersten Verbindungen sein.

Aufgrund der erfindungsgemäßen Lehre wird ein quasi schwebendes Sintern durchgeführt, wobei insbesondere der Träger, gegebenenfalls aber auch der Restrohling bzw. aus dem Rohling durch materialabtragende Bearbeitung hergestellte Abschnitte als Abstützmittel beim Sintern dienen.

In Weiterbildung der Erfindung ist vorgesehen, dass aus dem Rohling mehrere Formteile hergestellt werden, wobei vorzugsweise jedes Formteil über zumindest eine erste Verbindung wie einen Steg mit dem aus dem Restrohling ausgebildeten und zu diesem zumindest abschnittsweise beabstandeten Träger verbunden bleibt.

Sofern mehrere Formteile aus dem Rohling herausgearbeitet werden, besteht jedoch auch die Möglichkeit, dass die Formteile untereinander über Verbindungen wie Stege oder Membranen bzw. Abschnitte von Membranen verbunden sind und die Formteile insgesamt von einem Träger wie Rahmen zumindest abschnittsweise umgeben sind, der als Abstützung beim Sintern verwendet wird.

Insbesondere ist vorgesehen, dass als Rohling eine Scheibe verwendet wird, wobei eine Flachseite ein Abschnitt sein kann, auf dem der verbleibende Restrohling auf der Unterlage positioniert wird.

Vorzugweise wird jedoch der Träger, also der das Formteil umgebende Rahmen aus dem Restrohling gelöst, um sodann das Durchsintern durchzuführen. Dabei wird der Träger auf einer Unterlage positioniert. Ein unmittelbarer Kontakt zwischen dem Formling und der Unterlage erfolgt nicht, da der Formling im Bereich der Unterlage zurückversetzt zu dem Bereich verläuft, auf dem der Träger auf der Unterlage aufliegt.

Es besteht jedoch auch die Möglichkeit, einen Rohling in Zylinder- oder Quadergeometrie zu verwenden.

Besonders bevorzugt wird aus dem Rohling eine zumindest 7gliedrige zahntechnische Brücke als das Formteil hergestellt.

Des Weiteren bezieht sich die Erfindung auf einen Restrohling als Teil eines aus porösem keramischen Material bestehenden Rohlings mit einem aus dem Rohling durch Materialabtragung hergestellten Formteil, das integral mit dem Restrohling verbunden ist, und zeichnet sich dadurch aus, dass das Formteil über zumindest eine erste Verbindung mit einem durch Materialabtrag aus dem Rohling hergestellten und das Formteil zumindest abschnittsweise beabstandet umgebenden Träger verbunden ist, der seinerseits über zumindest eine zweite Verbindung beabstandet zu dem Restrohling mit diesem verbunden ist. Dabei verläuft das Formteil zurückversetzt zu einem als Abstützung auf einer Auflage dienenden Außenabschnitt des Trägers bzw. des Restrohlings.

Restrohling und Formteil sind integral aus dem Rohling hergestellt und über einen Träger verbunden, der gleichfalls aus dem Rohling hergestellt ist. Zwischen dem Formteil und dem Träger einerseits und diesem und dem Restrohling andererseits sind erste und zweite Verbindungen vorgesehen. Insoweit ist das Formteil integral mit dem Restrohling verbunden.

Zum Sintern wird jedoch bevorzugterweise der Restrohling von dem Träger getrennt, so dass die Einheit Träger und Formteil gesintert bzw. durchgesintert wird.

Insbesondere ist vorgesehen, dass der Träger das Formteil umlaufend beabstandet umgibt und über zumindest zwei erste Verbindungen wie Stege mit diesem verbunden ist. Vorzugsweise weist der Träger die Geometrie eines Rahmens auf, dessen Verlauf dem der Außenkontur des Formteils folgt.

Der Träger selbst ist über zumindest zwei erste und zwei zweite Verbindungen mit dem Formteil bzw. dem Restrohling verbunden, wobei die ersten und zweiten Verbindungen versetzt zueinander verlaufen.

Insbesondere ist vorgesehen, dass zumindest die ersten Verbindungen gleichmäßig entlang der Außenkontur des Formteils verteilt mit dem Träger verbunden sind.

Das Formteil ist vorzugsweise ein zahntechnisches Formteil wie eine mehrgliedrige, insbesondere zumindest 7gliedrige Brücke.

In Abhängigkeit von der Größe des Rohlings besteht des Weiteren die Möglichkeit, dass der Restrohling mehrere Formteile als integrale Bestandteile aufweist.

Anstelle von Stegen, die eine punktuelle Verbindung zwischen dem Formteil und dem Träger ermöglichen sollen, kann auch eine Verbindung in Art einer Membran durch Fräsen hergestellt werden, wobei die Membran umlaufend das Formteil oder aber abschnittsweise das Formteil mit dem Träger verbindet.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung eines der Zeichnung zu entnehmenden bevorzugten Ausführungsbeispiels.

Um ein Formteil wie zahntechnische Rekonstruktion herzustellen, werden im verstärkten Umfang Keramikmaterialien benutzt. Dabei werden Grünlinge oder vorgsinterte Rohlinge verwendet, da deren Bearbeitung relativ einfach ist. Nach Herstellung des Formteils muss dieses durchgesintert werden. Da das aus dem Rohling hergestellte Formteil um einen die nachfolgende Sinterschrumpfung exakt kompensierenden Vergrößerungsfaktor in allen Raumrichtungen linear vergrößert ist, ist sicherzustellen, dass beim Sintern ein Verzug nicht auftritt.

Um ein dimensionstreues Sintern zu ermöglichen, wird erfindungsgemäß der Rohling, bei dem es sich um einen Grünling oder einen vorgesinterten Körper aus einem porösen Keramikmaterial handeln kann, in eine Werkzeugmaschine eingespannt und sodann durch eine spanende Bearbeitung insbesondere durch Grob- und/ oder Feinfräsen herausgearbeitet.

Bei dem Rohling kann es ich um einen solchen handeln, der aus einem Metalloxidpulver aus der Gruppe Al₂O₃, TiO₂, MgO, Y₂O₃ und Zirkinoxidmischkristall Zr₁₋ₓMeₓO₂(4n/2)ₓ besteht, wobei Me ein Metall ist, das in Oxidform als zwei-, drei- oder vierwertiges Kation vorliegt und die tetragonale und/oder die kubische Phase des Zirkonoxids stabilisiert. Bei der Formel für Zirkonmischoxidkristall ist n = 2, 3 oder 4. Ferner gilt 0 ≤ x ≤ 1.

Die zum Fräsen benötigten Daten werden über die Steuerelektronik der Werkzeugmaschine übertragen und davon geeignete Werkzeugwege abgeleitet. Zur Gewinnung der Signale wird üblicherweise zuvor ein Modell digitalisiert oder es werden Daten aus einer Bibliothek entnommen. Insoweit wird auf hinlänglich bekannte Techniken verwiesen, wie diese auch z. B. in der WO-A-1999/47065 oder der WO-A-2002/45614 beschrieben sind. Auf die diesbezügliche Offenbarung wird ausdrücklich verwiesen.

Weitere Einzelheiten, Vorteile und Merkmale der Erfindung ergeben sich nicht nur aus den Ansprüchen, den diesen zu entnehmenden Merkmalen - für sich und/oder in Kombination -, sondern auch aus der nachfolgenden Beschreibung von der Zeichnung zu entnehmenden bevorzugten Ausführungsbeispielen.

Es zeigen:
- Fig. 1: einen Restrohling mit Formteil und Träger in Draufsicht,
- Fig. 2: einen Schnitt durch einen Restrohling mit Formteil und Träger entsprechend der Fig. 1,
- Fig. 3: einen Schnitt durch einen Restrohling mit einem Formteil und Träger nach einer weiteren Ausführungsform und
- Fig. 4: eine 7-gliedrige Brücke.

Anhand der Figuren soll verdeutlicht werden, dass aus einem vorzugsweise scheibenförmigem Rohling ein Formteil, im Ausführungsbeispiel eine mehrgliedrige Brücke 10, herstellbar ist, das anschließend durchgesintert werden kann, ohne dass die Gefahr eines Verzugs auftritt. Jedes Glied ist dabei als zahntechnische Einheit anzusehen.

Bei der nachfolgenden Beschreibung bevorzugter Ausführungsbeispiele sind grundsätzlich für gleiche Elemente gleich Bezugszeichen verwendet.

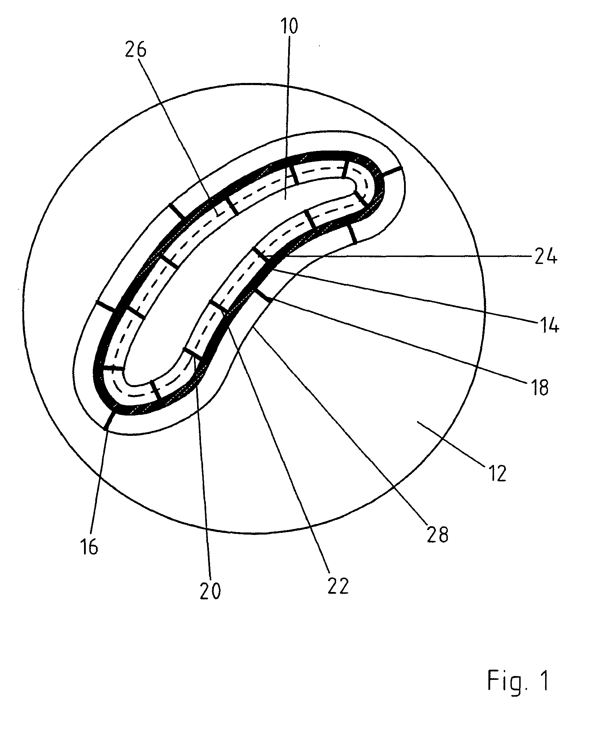

Dargestellt ist in Fig. 1 in Draufsicht ein Restrohling 12, also der Teil, der aus dem ursprünglichen Rohling verbleibt, nachdem das Formteil 10, ein dieses umgebender und einen Träger bildenden Rahmen 14 sowie den Rahmen 14 zum einen mit dem Formteil 10 und zum anderen mit dem Restrohling 12 verbindende Verbindungen durch spanende Bearbeitung herausgearbeitet worden sind. Der Rohling kann vorgesintert bzw. ein Grünling sein. Die Erfindung wird jedoch auch dann nicht verlassen, wenn ein Vorsintern nicht erfolgt.

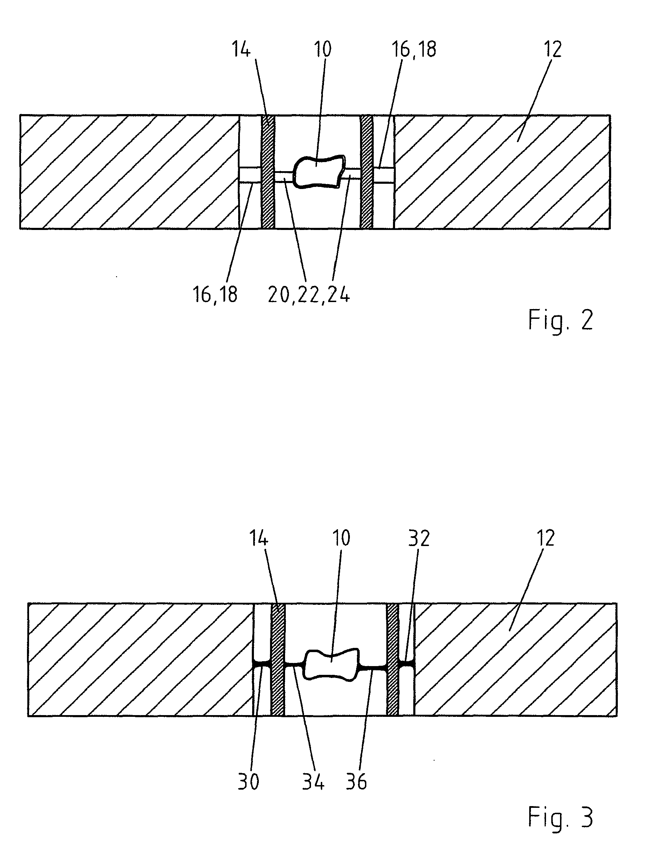

Im Ausführungsbeispiel der Fig. 1 und 2 sind die Verbindungen als Stege 16, 18, 20, 22, 24 ausgebildet. Dies ist jedoch nicht schutzeinschränkend zu verstehen. Verbindungen können auch durch umlaufende Membranen oder Abschnitte von Membranen oder Kombinationen von Membranen und Stegen oder gleichwirkenden Verbindungselementen verwirklicht werden, wie dies die Fig. 3 verdeutlichen soll. Aus Gründen der Vereinfachung wird nachstehend von Stegen gesprochen, gleichwenn erwähntermaßen hierunter auch andere in Frage kommende Verbindungen zu subsumieren sind.

Erfindungsgemäß werden zunächst das Formteil 10 und dann der Rahmen 14 durch Fräsen hergestellt, wobei die Stege 16, 18, 20, 22, 24 verbleiben, damit zumindest der Rahmen 14 und das Formteil 10 anschließend als Einheit gesintert werden können. Hierzu ist es zunächst erforderlich, dass der Rahmen 14 durch Durchtrennen der Stege 16, 18 aus dem Restrohling 12 herausgelöst wird.

Die Erfindung wird jedoch auch dann nicht verlassen, wenn der Restrohling 12 zusammen mit dem Rahmen 14 und dem Formteil 10 als Einheit gesintert werden würde.

Der Rahmen 14 kann die volle Höhe der Scheibe des Rohlings und damit des Restrohlings 12 aufweisen. Demgegenüber verlaufen die parallel zu den Flachseiten des Trägers oder des Rahmens 14 bzw. des Restrohlings 12 verlaufenden Seiten des Formteils 10 zu diesen beabstandet, bzw. es verläuft zumindest die Außenseite des Formteils 10, die der Seite zugewandt ist, auf der der Träger 14 oder der Restrohling 12 beim Sintern aufliegt, hierzu zurückversetzt.

Selbstverständlich kann auch der Rahmen 14 zurückversetzt sein, wenn ein Sintern zusammen mit dem Restrohling 12 durchgeführt wird.

Die Geometrie des Rahmens 14 berechnet sich wie folgt: Die Geometrie des Rahmens 14 wird errechnet ausgehend von der mittigen umlaufenden Bahn des Grobfräsers um die Formteilgeometrie; von dieser wird der Fräser um einen Wert errechnet aus Fräserradius + Distanzwert + Rahmendicke + Fräserradius umlaufend nach außen projiziert.

Die Breite des Rahmens 14 kann 1 mm bis 10 mm betragen, vorzugsweise jedoch 2 mm bis 3 mm. Die Höhe des Rahmens 14 umfasst die Höhe des verwendeten Rohlings und kann unter bestimmten frästechnischen Bedingungen zur Herstellung der Rekonstruktion auch davon abweichen.

Der Abstand zwischen Rahmen 14 und Rekonstruktion bzw. Formteil 10 sollte gleich doppelter Fräserradius plus einen Distanzwert von 0,1 mm bis 0,3 mm betragen.

Wie sich aus der Fig. 1 ergibt, sollten die zwischen dem Formteil 10, also der mehrgliedrigen Brücke, und dem Rahmen 14 verlaufenden Stege 16, 18, 20, 22, 24 gleichmäßig über den Umfang des Formteils 10 verteilt angeordnet sein. Dabei kann die Anzahl der Stege 20, 22, 24, über die das Formteil 10 mit dem Rahmen 14 verbunden ist, vorzugsweise größer als die Anzahl der Stege 16, 18 sein, die von dem Rahmen 14 ausgehen und in dem Rand 28 des Restrohlings 12 enden, der den Rahmen 14 umlaufend umgibt. Ferner sollten die Stege 16, 18 zwischen Rahmen 14 und Restrohling 12 zu den Stegen 20, 22, 24, die den Rahmen 12 mit dem Formteil 10 verbinden, versetzt zueinander verlaufen, um eine gewünschte relative Verschiebbarkeit zwischen Formteil 10, Rahmen 14 und Restrohling 12 zu ermöglichen, wodurch das dimensionstreue Sintern mit gewährleistet ist.

Der Abstand zwischen dem Rahmen 10 und dem umlaufenden Rand 28 des Rohlings ist in Abhängigkeit von der Geometrie der herzustellenden Rekonstruktion, also des Formteils zu wählen und kann zwischen 0 mm und 30 mm, vorzugsweise 0 mm und 3 mm betragen. Letzteres ermöglicht eine gute Ausnutzung der verfügbaren Rohlingsfläche. 0 mm bedeutet dabei, dass Außenrand des Trägers bzw. des Rahmens 14 gleich Abschnitt der Umfangsfläche des Rohlings selbst ist.

Hinsichtlich der Stege 16, 18, 20, 22, 24 ist anzumerken, dass diese einen runden oder ovalen Querschnitt aufweisen können. Der Querschnitt pro Steg sollte zwischen 0,5 mm² und 13 mm², vorzugsweise zwischen 1 mm² und 3 mm² liegen.

Die Anbringung der Stege 20, 22, 24 an das Formteil 10 sollte im größten Querschnitt der jeweiligen zahntechnischen Einheit erfolgen, jedoch nicht am Passungsrand einer jeweiligen Krone.

Die Anzahl der Stege beläuft sich pro zahntechnischer Einheit zwischen 0 bis 2, vorzugsweise 1. Bei einer mehrgliedrigen Brücke muss folglich nicht jedes Glied (zahntechnische Einheit) über einen Steg mit dem Rahmen verbunden sein. Besteht das Formteil 10 aus einer einzigen zahntechnischen Einheit, so beläuft sich die Anzahl der Stege auf zumindest 2.

Alternativ besteht die Möglichkeit, den Rahmen 14 mit dem Formteil 10 über eine Membran zu verbinden, die eine Dicke zwischen 0,1 mm und 1 mm, vorzugsweise im Bereich zwischen 0,3 mm und 0,5 mm aufweisen sollte. Alternativ können auch Verbindungen durch Teilbereiche einer Membran hergestellt werden, die in Umfangsrichtung des Formteils 10 Längen zwischen 0,5 mm und 5 mm, vorzugsweise zwischen 1 mm und 3 mm aufweisen sollten.

Durch die gewählte Konstruktion wird das Formteil 10 quasi schwebend gesintert.

Nach dem Durchsintern des Formteils 10 werden die Stege 20, 22, 24 von dem Formteil 10 getrennt und die Ansätze gegebenenfalls nachbearbeitet.

Ist im Ausführungsbeispiel aus dem Rohling ein einziges Formteil 10 herausgearbeitet, so besteht ohne Weiteres die Möglichkeit, mehrere Formteile gleichzeitig in dem Rohling herzustellen, die in zuvor beschriebener Weise über eine oder mehrere Verbindungen wie Stege mit Trägern verbunden sind, die ihrerseits beabstandet über weitere Verbindungen wie Stege zu dem verbleibenden Restrohling verlaufen. Dabei kann jedem Formteil ein gesonderter Rahmen oder mehreren Formteilen kann ein gemeinsamer Rahmen zugeordnet sein.

Aus der zeichnerischen Darstellung erkennt man, dass der Rahmen 14 im Wesentlichen parallel zur Außenkontur des Formteils 10 verläuft. Ferner sollte der Abstand zwischen dem Rahmen 14 und dem Formteil 10, also die einander zugewandten Bereiche einen Abstand aufweisen, der nicht viel größer als der wirksame Durchmesser des verwendeten Werkzeugs wie Fräsers ist. Gleiches gilt bezüglich des Abstands zwischen Umfangsfläche des Rahmens 14 und Begrenzungsrand 28 des Restrohlings 12. Der Rand 28 weist dabei einen Verlauf auf, der vorzugsweise der Außen- bzw. Umfangskontur des Rahmens 14 folgt.

Ist in dem Ausführungsbeispiel der Träger für das Formteil 10 als Rahmen 14 beschrieben worden, so soll hierdurch eine Beschränkung der Erfindung nicht erfolgen. Vielmehr sind auch andere Gestaltungen bzw. Geometrien möglich und somit von der Erfindung erfasst.

Die Fig. 2 entspricht dem Grunde nach der Fig. 1, stellt folglich einen Schnitt durch den Restrohling 12 dar, wobei jedoch bezüglich der Geometrien im Vergleich zu Fig. 1 Abweichungen bestehen. Ungeachtet sind die Stege 16, 18, 20, 22, 24 prinzipiell eingezeichnet, über die das Formteil 10 mit dem Träger beziehungsweise Rahmen 14 und dieser mit dem Restrohling 12 verbunden ist.

Aus einem Vergleich der Fig. 1 und 2 erkennt man, dass der Rohling eine Scheibe ist.

Wie zuvor erläutert worden ist, kann die Verbindung zwischen dem Formteil 10 und dem Träger 14 und zwischen diesem und dem Restrohling 12 nicht nur durch Stege 16, 18, 20, 22, 24, sondern auch durch Membranen oder Abschnitte von Membranen hergestellt werden, wie dies prinzipiell der Schnittdarstellung der Fig. 3 zu entnehmen ist. So ist das Formteil 10 über Membranen bzw. Abschnitte von Membranen 34, 36 mit dem Träger 14 verbunden, der seinerseits über Membranen 30, 32 oder Abschnitte dieser mit dem Restrohling 12 verbunden ist.

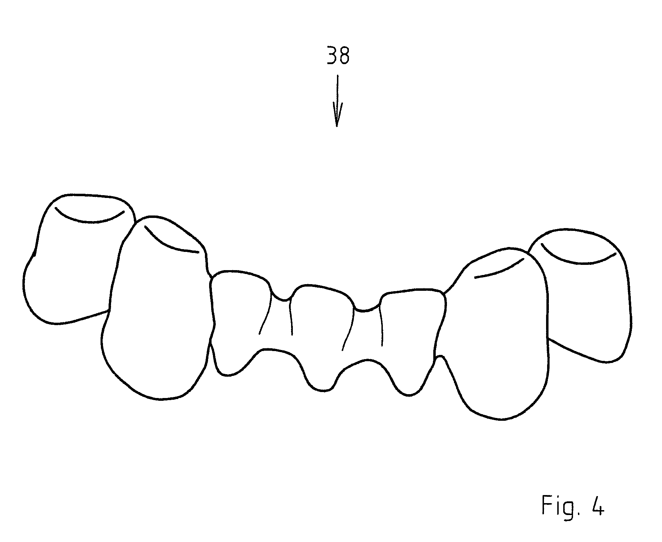

Des Weiteren ist der Fig. 4 eine 7-gliedrige Brücke 38 zu entnehmen, die unter Anwendung des erfindungsgemäßen Verfahrens hergestellt worden ist.

## Patentansprüche

1. Verfahren zum dimensionstreuen Sintern eines aus einem aus porösem keramischen Material bestehenden Rohling hergestellten Formteils (10), insbesondere zahntechnischer Rekonstruktion, wobei das Formteil während des Sinterns über zumindest eine erste Verbindung (20, 22, 24), wie Steg, mit einem Träger (14) verbunden bleibt, der aus dem Rohling hergestellt wird,
**dadurch gekennzeichnet,**
**dass** der Träger (14) als ein das Formteil (10) zumindest bereichsweise umlaufend umgebender Abschnitt des Rohlings durch materialabtragende Bearbeitung aus dem Rohling hergestellt wird, wobei nach der Herstellung der Träger zum einen über die zumindest eine erste Verbindung (20, 22, 24, 34, 36) mit dem Formteil und zum anderen über zumindest eine zweite Verbindung (16, 18, 30, 32) mit dem den Träger abschnittsweise beabstandet umgebenden Restrohling (12) verbunden ist.

2. Verfahren nach Anspruch 1,
**dadurch gekennzeichnet,**
**dass** der Träger (14) umlaufend das Formteil (10) umgibt und derart aus dem Rohling herausgearbeitet wie herausgefräst wird, dass der Träger zum einen über eine erste Membran (34, 36) oder Abschnitte dieser und/oder über zumindest zwei erste Stege (20, 22, 24) mit dem Formteil und zum anderen mit dem verbleibenden Restrohling (12) über eine zweite Membran (30, 32) oder Abschnitte dieser und/oder über zumindest zwei zweite Stege (16, 18) verbunden ist.

3. Verfahren nach Anspruch 1 oder 2,
**dadurch gekennzeichnet,**
**dass** zumindest das Formteil (10) zurückversetzt zu zumindest einem Abschnitt einer Außenfläche des Restrohlings (12) und/oder des Träger (14) verläuft, auf der der Restrohling beim Sintern auf einer Unterlage positioniert wird, wobei insbesondere sowohl das Formteil (10) als auch der Träger (14) zurückversetzt zu zumindest dem einen Abschnitt der Außenfläche des Restrohlings (12) verlaufen.

4. Verfahren nach zumindest einem der vorhergehenden Ansprüche,
**dadurch gekennzeichnet,**
**dass** vor dem Durchsintern der Träger (14) mit dem Formteil (10) von dem Restrohling (12) getrennt wird.

5. Verfahren nach zumindest einem der vorhergehenden Ansprüche,
**dadurch gekennzeichnet,**
**dass** der Träger (14) in seiner Höhe unbearbeitet bleibt.

6. Verfahren nach zumindest einem der vorhergehenden Ansprüche,
**dadurch gekennzeichnet,**
**dass** aus dem Rohling mehrere Formteile (10) hergestellt werden, wobei vorzugsweise jedes Formteil über zumindest eine erste Verbindung (16, 18) wie Steg und/oder Membran mit dem aus dem Restrohling (12) ausgebildeten Träger (14) verbunden bleibt, wobei gegebenenfalls jedes Formteil (10) von einem gesonderten Träger (14) oder mehrere Formteile (10) von einem gemeinsamen Träger (14) umgeben werden.

7. Verfahren nach zumindest einem der vorhergehenden Ansprüche,
**dadurch gekennzeichnet,**
**dass** das Formteil (10) von einem Rahmen als Träger (14) umgeben wird, dessen Verlauf Umfangskontur des Formteils folgt, wobei vorzugsweise als Rohling eine Scheibe verwendet wird.

8. Verfahren nach zumindest einem der vorhergehenden Ansprüche,
**dadurch gekennzeichnet,**
**dass** aus dem Rohling eine zumindest 7-gliedrige zahntechnische Brücke (38) als das Formteil (10) hergestellt wird.

9. Restrohling (12) als Teil eines aus porösen keramischen Material bestehenden Rohlings mit zumindest einem aus dem Rohling durch Materialabtragung hergestellten Formteil (10), das integral mit dem Restrohling ausgebildet ist,
**dadurch gekennzeichnet,**
**dass** das Formteil (10) über zumindest eine erste Verbindung (20, 22, 24, 34, 36) mit einem durch Materialabtragung aus dem Rohling hergestellten und das Formteil zumindest abschnittsweise beabstandet umgebenden Träger (14) verbunden ist, der seinerseits über zumindest eine zweite Verbindung (16, 18, 30, 32) beabstandet zu dem Restrohling (12) mit diesem verbunden ist.

10. Restrohling nach Anspruch 9,
**dadurch gekennzeichnet,**
**dass** zumindest das Formteil (10) zu einem als Abstützung auf einer Auflage dienenden Außenabschnitt des Trägers (14) und/oder des Restrohlings (12) zurückversetzt verläuft, insbesondere sowohl das Formteil (10) als auch der Träger (14) zu dem Außenabschnitt des Restrohlings (12) zurückversetzt verläuft.

11. Restrohling nach einem der Ansprüche 9 oder 10,
**dadurch gekennzeichnet,**
**dass** der Träger (14) das Formteil (10) umlaufend umgibt und über zumindest zwei erste Verbindungen (20, 22, 24, 34, 36) wie Stege und/oder zumindest abschnittsweise über eine aus dem Rohling herausgearbeitete Membran mit dem Formteil und/oder dass der Träger (14) über zumindest zwei zweite Verbindungen (16, 18, 30, 32) wie Stege und/oder eine Membran mit dem Restrohling (12) verbunden ist, wobei vorzugsweise die ersten Verbindungen zu den zweiten Verbindungen versetzt angeordnet sind.

12. Restrohling nach einem der Ansprüche 9 bis 11,
**dadurch gekennzeichnet,**
**dass** der Abstand zwischen dem Formteil (10) und dem Träger (14) in etwa gleich wirksamer Durchmesser des das Formteil aus dem Rohling herausarbeitenden Werkzeugs ist und/oder dass der Abstand zwischen dem Träger (14) und diesen umgebenden Rand (28) des Restrohlings (12) zumindest abschnittsweise in etwa wirksamer Durchmesser des den Träger herausarbeitenden Werkzeugs ist.

13. Restrohling nach einem der Ansprüche 9 bis 12,
**dadurch gekennzeichnet,**
**dass** der Träger (14) ein Rahmen ist, dessen Geometrie der Außenkontur des Formteils (10) folgt und/oder dass der den Träger (14) umgebende zugewandte Rand (28) des Restrohlings (12) zumindest abschnittsweise der Umfangskontur des Trägers folgt.

14. Restrohling nach einem der Ansprüche 9 bis 13,
**dadurch gekennzeichnet,**
**dass** Außenrand des Trägers (14) abschnittsweise ein Abschnitt des Außenrands des Rohlings ist.

15. Restrohling nach einem der Ansprüche 9 bis 14,
**dadurch gekennzeichnet,**
**dass** das Formteil (10) ein zahntechnisches Formteil, vorzugsweise eine zumindest 7-gliedrige Brücke (38) ist.
